# EUROPÄISCHE PATENTSCHRIFT

(11) **EP 2 720 042 B1**
(45) Veröffentlichungstag und Bekanntmachung des Hinweises auf die Patenterteilung: **20.07.2016**
(21) Anmeldenummer: 13004717.8
(22) Anmeldetag: 27.09.2013
(51) Int. Cl.: B01J 20/28, G01N 33/543, B01J 20/291

(54) **Verfahren zur Herstellung von porösen Gelen mit inkorporierten katalytisch oder biologisch aktiven Materialien sowie damit hergestellte Gele und deren Verwendung**
Method for the preparation of porous gels with incorporated catalytic or biologically active materials, gels produced with same and the utilisation thereof
Procédé de fabrication de gels poreux à l'aide de matériaux incorporés actifs au niveau catalytique ou biologique, gels fabriqués à l'aide de ce procédé et leur utilisation

(30) Priorität: 11.10.2012 DE 102012019984
(43) Veröffentlichungstag der Anmeldung: 16.04.2014
(73) Patentinhaber: Leibniz-Institut für Oberflächenmodifizierung e.V., 04318 Leipzig (DE)
(72) Erfinder: Abel, Bernd, 04416 Markkleeberg (DE); Reichelt, Senta, 01157 Dresden (DE); Elsner, Christian, 04249 Leipzig (DE)
(74) Vertreter: Katzameyer, Michael

(56) Entgegenhaltungen:
- CN-A- 102 492 684
- KUMAKURA M: "PREPARATION METHOD OF POROUS POLYMER MATERIALS BY RADIATION TECHNIQUE AND ITS APPLICATION", POLYMERS FOR ADVANCED TECHNOLOGIES, WILEY & SONS, BOGNOR REGIS, GB, Bd. 12, Nr. 7, 1. Juli 2001 (2001-07-01), Seiten 415-421, XP001102921, ISSN: 1042-7147, DOI: 10.1002/PAT.69
- PLIEVA F M ET AL: "Cryogel applications in microbiology", TRENDS IN MICROBIOLOGY, ELSEVIER SCIENCE LTD., KIDLINGTON, GB, Bd. 16, Nr. 11, 1. November 2008 (2008-11-01), Seiten 543-551, XP025608827, ISSN: 0966-842X, DOI: 10.1016/J.TIM.2008.08.005 [gefunden am 2008-10-04]

## Beschreibung

### Hintergrund der Erfindung

Die vorliegende Erfindung betrifft Verfahren zur Herstellung von porösen Gelen, insbesondere Cryogelen, mit inkorporierten katalytisch oder biologisch aktiven Materialien sowie damit hergestellte Gele und deren Anwendungen auf verschiedenen Gebieten.

Poröse Gele werden seit längerem als Trägersysteme für katalytisch und/oder biologisch aktive Materialien auf verschiedensten Gebieten, beispielsweise in der Katalysatortechnik, der Mikrobiologie, und der Analytik, einschließlich der medizinischen Diagnostik, verwendet.

Zur Herstellung solcher porösen Gele wurden sehr unterschiedliche Ansätze verfolgt und in den letzten Jahren erhielten insbesondere Techniken, die auf der Bildung von sogenannten Cryogelen beruhen, große Aufmerksamkeit.

Ein Cryogel entsteht üblicherweise durch Bereitstellung eines flüssigen Mediums, das gewünschte Monomerverbindungen und Polymerisationsinitiatoren in einem geeigneten Lösungsmittel enthält, Abkühlung des flüssigen Mediums unterhalb des Gefrierpunkts des Lösungsmittels in diesem System, wodurch eine strukturierte feste Phase entsteht, Polymerisation bei dieser tiefen Temperatur, wobei die beim Abkühlen gebildete feste Phase als Matrize für das entstehende Polymerisat dient, und Entfernen des Lösungsmittels, wodurch ein poröses Gel entsteht, dessen Poren eine durch die Strukturen der vorübergehend anwesenden festen Phase des Lösungsmittels vorgegebene Größe und Form aufweisen. In einer Variation dieses Verfahrens wird die Polymerisation auch in Gegenwart von biologisch aktiven Materialien wie z.B. Zellen durchgeführt (Plieva et al., Trends in Microbiology, (2008), Bd. 16, Nr. 543-551).

Nachteilig bei diesen herkömmlichen Verfahren ist die Anwesenheit von nicht verbrauchten Polymerisationsinitiatoren und deren Fragmenten und häufig Resten von organischen Lösungsmitteln in den entstandenen Cryogelen. Diese organischen Kontaminationen sind oft toxisch bzw. schädlich für biologische Zellen und andere biologisch aktive Materialien. Ein weiterer Nachteil sind die typischerweise langen Reaktionszeiten (in der Größenordnung von durchschnittlich 15 Stunden), und die Tatsache, dass die Polymerisationsreaktion in der Regel bereits vor dem Einfrieren beginnt.

Zur Vermeidung der letzteren Nachteile kann eine photoinitiierte radikalische Polymerisation durchgeführt werden. Das dabei üblicherweise verwendete UVA-Licht mit einer Wellenlänge von 310-390 nm besitzt jedoch nur eine sehr begrenzte Eindringtiefe und die Reaktionsgefäße müssen UVtransparent sein. Außerdem ist auch diese Methode auf die Verwendung meist toxischer Initiatoren angewiesen. Ein solches Verfahren wird beispielsweise in der chinesischen Patentanmeldung CN 102 492 684 A beschrieben.

Ein Verfahren zur Herstellung poröser Gele, das ohne organische Polymerisationsinitiatoren auskommt, beruht auf einer Gammastrahlen-induzierten Polymerisation in Gegenwart von Urease bei tiefen Temperaturen und wurde von M. Kumakura in Polym. Adv. Technol. 12, 415-421 (2001) beschrieben. Die resultierenden kleinporigen Gele (Porengrößen unterhalb von 30 µm) hatten jedoch nur eine geringe mechanische Festigkeit, sodass zur besseren Handhabung der Gele der Zusatz eines Verstärkungsfasermaterials (AT-200) erforderlich war und die Enzymeinlagerung erst bei relativ hohen Monomerkonzentrationen ab 20 % (nur geringe Restaktivität des Enzyms von etwa 20 %, mit einem Optimum der Monomerkonzentration bei etwa 70 %) möglich wurde. Der hohe Acrylatanteil wäre sehr nachteilig für die Anwendung mit lebenden Organismen.

Die dort verwendeten Bestrahlungszeiten liegen in der Größenordnung von Stunden und sind für den praktischen Einsatz nicht optimal. Darüber hinaus ist dieses Verfahren auch aufgrund der Verwendung von Gammastrahlen kaum zur Herstellung von Gelen in Gegenwart von lebenden Zellen geeignet.

Ein weiterer Nachteil besteht in der Verwendung radioaktiver Quellen und den damit verbundenen Problemen (Sicherheit, Entsorgung radioaktiven Materials).

Eine Aufgabe der Erfindung ist die Bereitstellung eines neuen Verfahrens zur Herstellung poröser Gele mit inkorporierten biologisch aktiven Substanzen, mit welchem die oben aufgeführten Nachteile herkömmlicher Verfahren ganz oder weitgehend vermieden werden. Eine weitere Aufgabe der Erfindung ist die Bereitstellung neuartiger poröser Gele, welche frei von organischen Lösungsmitteln und Polymerisationsinitiatoren sind.

Überraschend konnten diese Aufgaben durch die Bereitstellung des erfindungsgemäßen Verfahrens nach Anspruch 1 und die Bereitstellung des porösen Gels nach Anspruch 8 gelöst werden. Speziellere Aspekte und bevorzugte Ausführungsformen der Erfindung sind Gegenstand der weiteren Ansprüche.

### Beschreibung der Erfindung

Das erfindungsgemäße Verfahren zur Herstellung poröser Gele, die Poren im Nano- bis Mikrometerbereich aufweisen, mit inkorporierten katalytisch oder biologisch aktiven Substanzen umfasst mindestens die folgenden Schritte:
a) Bereitstellen eines flüssigen Mediums, welches mindestens ein Lösungsmittel oder ein Lösungsmittelgemisch, das bei Abkühlung unter eine Phasenübergangstemperatur eine feste Phase mit amorphen und/oder kristallinen Strukturen ausbildet, mindestens eine durch Elektronenstrahlung oder kurzwellige UV-Strahlung im Wellenlängenbereich von 100 bis 250 nm vernetzbare Monomerkomponente, sowie mindestens ein katalytisch oder biologisch aktives Material in Lösung, Dispersion oder Emulsion umfasst und frei von Polymerisationsinitiatoren ist;
b) Abkühlen des flüssigen Mediums auf eine Temperatur unterhalb der Phasenübergangstemperatur des flüssigen Mediums;
c) Bestrahlen des abgekühlten Mediums von Schritt b) mit Elektronenstrahlung oder kurzwelliger UV-Strahlung im Wellenlängenbereich von 100 nm bis 250 nm mit einer ausreichenden Dosis, um eine Vernetzung der vernetzbaren Monomerkomponente(n) zu erreichen, wobei die amorphen oder kristallinen Strukturen der in Schritt b) gebildeten festen Phase als Matrize (Template) für das entstehende Netzwerk dienen;
d) Entfernen des Lösungsmittels, wodurch ein poröses Gel entsteht, dessen Poren eine durch die amorphen oder kristallinen Strukturen der in Schritt b) gebildeten festen Phase vorgegebene Größe und Form aufweisen.

In dem erfindungsgemäßen Verfahren ist das flüssige Medium frei von Polymerisationsinitiatoren. Durch die erfindungsgemäße Bestrahlung mit Elektronenstrahlung oder kurzwelliger UV-Strahlung im Wellenlängenbereich von 100 nm bis 250 nm, vorzugsweise 100-230 nm oder 120-230 nm, insbesondere 172-222 nm, kann eine ausreichende Vernetzung der Monomerkomponente(n) bereits in Abwesenheit der üblichen Polymerisationsinitiatoren erfolgen.

Zur Bestrahlung mit Elektronenstrahlung kann beispielweise ein im Stand der Technik bekannter Linearbeschleuniger (z.B. wie von Toray Company, Russland, im Handel erhältlich) eingesetzt werden.

Zur Bestrahlung mit kurzwelliger UV-Strahlung ist grundsätzlich jede Strahlungsquelle geeignet, welche Photonen mit der gewünschten Wellenlänge erzeugt. Bevorzugte Strahlungsquellen sind Excimerlampen (mit Edelgas und Edelgas/Halogen-Gemischen, vorzugsweise Xe₂*, Kr₂*, Ar₂*, KrCl*) und Excimerlaser, insbesondere Argonlaser, Halogenlaser und Argonhalogenidlaser, und Plasmen, vorzugsweise Atmosphärendruckplasmen.

Die Vernetzung durch Elektronenstrahlung erfolgt typischerweise bei einer Dosis von 2 bis 150 kGy, vorzugsweise von 5 bis 50 kGy, für einen Zeitraum von 1 bis 30 min, vorzugsweise von 5 bis 15 min.

Die Vernetzung durch kurzwellige UV-Strahlung wird in der Regel bei einer Dosis im Bereich von >0.1 mJ/cm², vorzugsweise 1-1000 mJ/cm², erfolgen. Ein typischer Fall ist eine UV-Polymerisation, bei der die Probe mit Hilfe einer Bandanlage durch die Bestrahlungszone einer 222nm-Excimerlampe (KrCl*) mit einer Leistung von 100 mW/cm² bei einer Bandgeschwindigkeit von 5 m/min gefahren wird. Die Bestrahlungszeit liegt hierbei typischerweise in einem Zeitraum von wenigen Sekunden bis Millisekunden. In einem anderen Fall (siehe Beispiel 6) kann die Bestrahlung auch stationär erfolgen, die Bestrahlungszeiten sind dementsprechend länger, typischerweise im Bereich bis zu 5 min, z.B. 10 s bis 3 min oder 10 s bis 2 min.

Die jeweils verwendete Dosis muss hoch genug sein, um die gewünschte Vernetzung zu erreichen, und so niedrig, dass das jeweilige (bio)aktive Material nicht oder nicht erheblich geschädigt wird. Geeignete Dosen können jedoch vom Fachmann unschwer mit Routineversuchen ermittelt werden.

Erfindungsgemäß verwendete Lösungsmittel können Wasser, ein organisches Lösungsmittel mit einem Phasenumwandlungspunkt im Bereich von -100°C bis +100°C, z.B. DMSO, Dioxan, tert. Butanol, langkettige Alkohole mit mehr als 5 C-Atomen, z.B. 6-20 C-Atomen, Öle, (geschmolzene) Wachse etc., eine ionische Flüssigkeit, z.B. auf der Basis von organischen Imidazolium-, Pyridininum-, Pyrrolidinium-, Guanidinium, Uronium-, Thiouronium-, Piperidinium-, Morpholinium-, Ammonium- oder Phosphonium-Kationen und organischen oder anorganischen Anionen wie z.B. Halogeniden, Tetrafluoroboraten, Trifluoracetaten, Phosphinaten, Tosylaten, Imiden und Amiden, oder superkritische Fluide, wie z.B. CO₂, Wasser, Propan und zahlreiche weitere Kohlenwasserstoffe in einem superkritischen Zustand, umfassen oder daraus bestehen.

Das flüssige Medium kann auch eine Mischung verschiedener Lösungsmittel, z.B. eine Mischung von Wasser mit einem organischen Lösungsmittel etc., umfassen. Diese verschiedenen Lösungsmittel können auch unterschiedliche feste Phasen nebeneinander ausbilden.

Geeignete Lösungsmittel oder Lösungsmittelmischungen für gewünschte Monomerkomponenten kann der Fachmann unschwer anhand seines Fachwissens und/oder durch Routineversuche ermitteln.

In einer spezielleren Ausführungsform umfasst das erfindungsgemäße Verfahren die folgenden Schritte:
a) Bereitstellen eines flüssigen Mediums, welches als Lösungsmittel Wasser, mindestens eine durch Elektronenstrahlung oder kurzwellige UV-Strahlung im Wellenlängenbereich von 100 nm bis 250 nm, vorzugsweise 100 nm bis 230 nm, besonders bevorzugt 172 nm bis 222 nm, vernetzbare Monomerkomponente, sowie mindestens ein katalytisch oder biologisch aktives Material in Lösung, Dispersion oder Emulsion umfasst und frei von Polymerisationsinitiatoren ist;
b) Abkühlen des flüssigen Mediums auf eine Temperatur unterhalb des Gefrierpunkts, vorzugsweise im Bereich zwischen -15°C und -30°C,
c) Bestrahlen des abgekühlten, gefrorenen Mediums von Schritt b) mit Elektronenstrahlung oder kurzwelliger UV-Strahlung im Wellenlängenbereich von 100 nm bis 250 nm, vorzugsweise 100 nm bis 230 nm, besonders bevorzugt 172 nm bis 222 nm, mit einer ausreichenden Dosis, um eine Vernetzung der vernetzbaren Monomerkomponente(n) zu erreichen;
d) Entfernen des Lösungsmittels.

Die Entfernung des mindestens einen Lösungsmittels kann mit jedem geeigneten Verfahren, vorzugsweise durch Verdampfung, Sublimation oder Ausspülen, erfolgen.

Als Monomerkomponente ist grundsätzlich jede durch Elektronenstrahlung oder kurzwellige UV-Strahlung mit einer Wellenlänge im Bereich von 100-250 nm, vorzugsweise 100 nm bis 230 nm, besonders bevorzugt 172 nm bis 222 nm, vernetzbare Monomerverbindung geeignet.

Typischerweise weist die mindestens eine vernetzbare Monomerkomponente mindestens eine polymerisierbare Doppelbindung auf. Es können jedoch Monomerkomponenten ohne Doppelbindungen ebenfalls vernetzt werden.

Der Begriff "vernetzbare Monomerkomponente" wie hier verwendet, soll ausdrücken, dass die betreffende Verbindung einer Polymerisations-, Additions-, Kondensations- oder anderen Verknüpfungsreaktion zugänglich ist, die zu einem makromolekularen Produkt führt.

In einer spezielleren Ausführungsform ist die mindestens eine vernetzbare Monomerkomponente aus der Gruppe aus Acrylaten, Methacrylaten, Acrylamiden, Methacrylamiden, Styrolverbindungen, Vinylverbindungen, Allylverbindungen, Dienen, Epoxiden und deren Derivaten ausgewählt.

Diese Derivate sind oder umfassen insbesondere Konjugate von Acrylaten, Methacrylaten, Acrylamiden, Methacrylamiden, Styrolverbindungen, Vinylverbindungen, Allylverbindungen, Dienen, Epoxiden mit anderen Molekülen und Makromolekülen, insbesondere Polysachariden, z.B. Cellulose, Chitin, Alginate, Hyaluronsäure etc., Polyaminosacchariden, z.B. Chitosan; Proteinen, z.B. Collagen, Gelatine; Lipiden; anderen vernetzbaren Biopolymeren, und Liposomen/Mizellen.

Das flüssige Medium wird neben mindestens einer vernetzbaren Haupt-Monomerkomponente wie oben definiert in der Regel auch noch mindestens eine zusätzliche multifunktionelle Vernetzerverbindung enthalten, um den Vernetzungsgrad zu erhöhen.

Solche Vernetzer können prinzipiell alle für die jeweilige Haupt-Monomerkomponente(n) als geeignet bekannten Vernetzermoleküle sein. Der Vernetzer kann beispielsweise aus der Gruppe aus Acrylaten, Methacrylaten, Acrylamiden, Methacrylamiden, Styrolverbindungen, Vinylverbindungen, Allylverbindungen, Dienen, Epoxiden und deren Derivaten ausgewählt sein. In einer speziellen Ausführungsform kann der Vernetzer beispielsweise Tetraethylenglykoldiacrylat oder -dimethacrylat sein.

Die vernetzbaren Monomerkomponente(n) (einschließlich Vernetzer) liegen typischerweise in einer Gesamtmenge von 1-20 Gew.-% oder 4-19 Gew.-%, vorzugsweise 1-15 Gew.-%, insbesondere 4-10 Gew.-% oder 4-9 Gew.-%, in dem flüssigen Medium vor.

Das Verhältnis des multifunktionellen Vernetzers zu dem oder den anderen Monomer(en) kann im Bereich von Vernetzer:Monomer 30:0 bis 2:28 liegen. Typischerweise liegt das Gewichtsverhältnis von Vernetzer:Monomer im Bereich von 10:1 bis 1:10, bevorzugter 5:1 bis 1:5. Besonders bevorzugt ist ein Gewichtsverhältnis von ca. 1:1 (bzw. falls drei Komponenten enthalten sind, von 1:1:1).

Ein besonderer Vorteil des erfindungsgemäßen Verfahrens liegt darin, dass bereits bei relativ niedrigen Gesamtmonomerkonzentrationen von 10 % (oder sogar weniger) die stabile Einlagerung von aktiven Materialien möglich ist und beispielsweise Enzyme mit sehr hoher Aktivät nach der Einlagerung unter diesen Bedingungen eingelagert werden können.

Ein Polymerisationsinitiator ist allgemein eine chemische Verbindung, welche mindestens eine funktionelle Gruppe trägt, die durch Änderung eines physikalisch-chemischen Parameters (ein Signal) entsprechend reaktive Spezies erzeugt, die eine Polymerisation initiieren, jedoch wobei die funktionelle Gruppe bzw. Fragmente hiervon nicht weiter in den maßgeblichen Kettenwachstumsprozess des Polymers während der Polymerisationsreaktion involviert ist/sind. Der physikalisch-chemische Parameter kann z.B. Licht, thermische Energie oder eine pH-Änderung sein.

Ein Polymerisationsinitiator wie hier betrachtet ist insbesondere ein Photoinitiator (z.B. Typ I Photoinitiatoren: u.a. Benzoinderivate, Acetophenonderivate, Benzilketale, Alpha-Hydroxyalkylphenone und Alpha-Aminoalkylphenone, O-Acyl-alphaoximinoketone, Acylphoshinoxide und Acylphosphonate, Peroxyverbindungen, etc.; Typ II Photoinitiatoren: u.a. Benzophenone, Xanthone und Thioxanthone, Alpa-Ketokumarine" aromatische 1,2-Diketone, Phenylglyoxilate)im speziellen Fall z.B. Benzoylbenzyl)trimethylammoniumchlorid und Lucirin TPO bzw. Lucirin TPO-L, d.h. eine chemische Verbindung, insbesondere organische Verbindung, die mindestens eine chromophore Gruppe enthält, welche bei der Bestrahlung mit Photonen entsprechend reaktive Spezies (z.B. Radikale, reaktive Ionen etc.) erzeugt, die eine Polymerisation initiieren, jedoch deren chromophore Gruppe darüber hinaus nicht weiter in den maßgeblichen Kettenwachstumsprozess der Polymerisation involviert ist.

Ein Polymerisationsinitiator ist z.B. auch ein thermischer Initiator, der mindestens eine funktionelle Gruppe enthält, welche bei Zufuhr thermischer Energie entsprechend reaktive Spezies erzeugt, die eine Polymerisation initiieren, jedoch dessen funktionelle Gruppe darüber hinaus nicht weiter in den maßgeblichen Kettenwachstumsprozess der Polymerisation involviert ist (beispielsweise N,N,N',N'-Tetramethylethylendiamin/Ammoniumpersulfat).

Ein erfindungsgemäß verwendetes flüssiges Medium, das "frei von Polymerisationsinitiatoren ist", ist somit ein flüssiges Medium wie oben definiert, welches ein Lösungsmittel oder ein Lösungsmittelgemisch, das bei Abkühlung unter eine Phasenübergangstemperatur eine feste Phase mit amorphen und/oder kristallinen Strukturen ausbildet, mindestens eine durch Elektronenstrahlung oder kurzwellige UV-Strahlung im Wellenlängenbereich von 100 bis 250 nm vernetzbare Monomerkomponente, sowie mindestens ein katalytisch oder biologisch aktives Material in Lösung, Dispersion oder Emulsion umfasst, und darüber hinaus keine Additive enthält, welche als Polymerisationsinitiatoren wie oben definiert für die jeweilige Vernetzungsreaktion wirken, insbesondere keine Polymerisationsinitiatoren wie oben beispielhaft erwähnt.

Die erfindungsgemäß verwendeten katalytisch oder biologisch aktiven Materialien sind nicht besonders beschränkt und grundsätzlich kommen alle Materialien in Frage, die unter den verwendeten Vernetzungsbedingungen stabil bleiben.

In einer spezielleren Ausführungsform sind diese Materialien aus der Gruppe ausgewählt, die Katalysatoren, physiologische oder pharmazeutische Wirkstoffe (z.B. Antibiotika), Aminosäuren, Peptide, Oligopeptide, Proteine, Enzyme, Nukleotide, Nukleoside, Nukleinsäuren (DNA,RNA), Ribozyme, Saccharide, Oligosaccharide, Polysaccharide, Lipide, Membranen und Membrankomponenten, intra- und interzelluläre Regulationsstoffe (z.B. Hormone), Viren, Zellbestandteile, Zellorganellen, Zellen, Zellverbände, Gewebe in ihrer natürlichen oder artifiziell konstruierten oder modifizierten Form umfasst.

Der Begriff "Zellen", wie hier verwendet, umfasst sowohl prokaryotische als auch eukaryotische Zellen, insbesondere Zellen von Bakterien und Archaebakterien, Pilzzellen, tierische und pflanzliche Zellen. Die Zellen können sowohl natürliche Zellen sein als auch Zellen, die in irgendeiner Weise, z.B. gentechnisch, modifiziert wurden. Diese Modifizierung kann insbesondere die Einführung zusätzlicher Zellbestandteile, (z.B. zusätzliches Genmaterial), den Austausch natürlicher Zellbestandteile durch entsprechende Bestandteile aus anderen Zellen oder künstliche Konstrukte, und die Deletion bestimmter Zellbestandteile, sowie Kombinationen davon umfassen.

Diese Materialien können insbesondere auch völlig synthetisch aufgebaute artifizielle Zellen und Vesikel umfassen, die Komponenten von Viren und Zellen, Aminosäuren, Peptide, Oligopeptide, Proteine, Enzyme, Nukleotide, Nukleoside, Nukleinsäuren (DNA,RNA), Ribozyme, Saccharide, Oligosaccharide, Polysaccharide, Lipide, Membranen und Membrankomponenten umfassen oder daraus bestehen.

In einer besonders bevorzugten Ausführungsform sind die Zellen lebende prokaryotische oder eukaryotische Zellen.

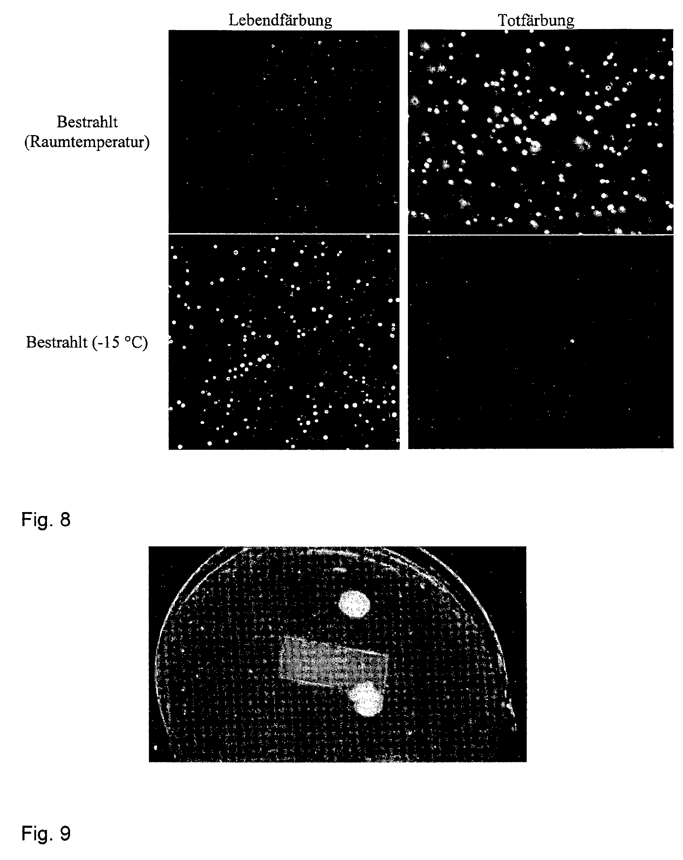

Es war besonders überraschend, dass die Elektronenbestrahlung biologischen Materials, insbesondere lebender Zellen, in gefrorenen wässrigen Systemen, nicht wie erwartet zu einem signifikanten Verlust der biologischen Aktivität (Sterilisation) führte, wie dies üblicherweise bei einer Bestrahlung in flüssigen wässrigen Medien zu beobachten ist. Die Figuren 8 und 9 sowie Beispiel 4 zeigen dies für eine Bestrahlung bei ca. -15°C bis -20°C. Jedoch sind auch tiefere Temperaturen wie z.B. -30°C oder die Temperatur von flüssigem Stickstoff grundsätzlich möglich.

Mit dem erfindungsgemäßen Verfahren können sowohl Gelpartikel als auch flächige oder dreidimensionale Strukturen, letztere hier als "monolithische Strukturen" bezeichnet, hergestellt werden.

Die Ausführungsform des erfindungsgemäßen Verfahrens bei der kurzwellige UV-Strahlung verwendet wird, eignet sich aufgrund der geringeren Eindringtiefe der Photonen in Materialien vorzugsweise zur Herstellung von entsprechenden Gelen auf flächigen Substraten.

Das erfindungsgemäße Verfahren zur Herstellung von Gelpartikeln umfasst typischerweise eine Emulsionspolymerisation von in dem jeweiligen Lösungsmittel unlöslichen vernetzbaren Monomerkomponenten.

Bei dieser Emulsionspolymerisation kann die Tröpfchenbildung durch Ultraschall, Dispergieren bei hohen Drehzahlen oder Pressen der Emulsion durch einen Filter oder andere gängige Verfahren gefördert werden.

Eine spezielle Ausführungsform des erfindungsgemäßen Verfahrens ist dadurch gekennzeichnet, dass das flüssige Medium ferner eine Reporterkomponente umfasst, welche das Erreichen eines gewünschten Vernetzungsgrads durch eine nachweisbare Änderung eines physikalischen Parameters, vorzugsweise eines optischen Parameters wie Farbe oder Fluoreszenz, anzeigt.

Ein weiterer Aspekt der Erfindung betrifft ein poröses Gel mit Poren im Mikrometerbereich, welches mit dem erfindungsgemäßen Verfahren erhältlich ist und eine vernetzte Polymerkomponente und inkorporierte katalytisch oder biologisch aktive Materialien umfasst, dadurch gekennzeichnet, dass es frei von Polymerisationsinitiatoren und organischen Lösungsmitteln ist und eine mittlere Porengröße von 30-100 µm, vorzugsweise 35-100 µm, aufweist.

Ein erfindungsgemäßes Gel, das "frei von Polymerisationsinitiatoren ist", ist ein Gel, das, außer eventuellen Resten von nicht umgesetzten Monomerkomponenten und Vernetzern, keine weitere Substanz, insbesondere keine organische Substanz enthält, welche für die jeweilige Polymerisationsreaktion, die zur Bildung des Gels führte, als Initiator, insbesondere Photoinitiator bzw. thermischer Initiator, dienen kann.

Der Begriff "poröses Gel", wie hier verwendet, bezieht sich auf eine dreidimensional vernetzte polymere Gerüststruktur mit verbundenen Poren im Nano- bis Mikrometerbereich.

Die mittlere Porengröße der erfindungsgemäßen Gele liegt im Bereich von 30-100 µm, bevorzugter 35-100 µm oder 50-100 µm.

Diese Gele können grundsätzlich beliebige katalytisch oder biologisch aktive Materialien umfassen.

In einer spezielleren Ausführungsform sind diese Materialien aus der Gruppe ausgewählt, die Katalysatoren, physiologische oder pharmazeutische Wirkstoffe (z.B. Antibiotika), Aminosäuren, Peptide, Oligopeptide, Proteine, Enzyme, Nukleotide, Nukleoside, Nukleinsäuren (DNA,RNA), Ribozyme, Saccharide, Oligosaccharide, Polysaccharide, Lipide, Membranen und Membrankomponenten, intra- und interzelluläre Regulationsstoffe (z.B. Hormone), Viren, Zellbestandteile, Zellorganellen, Zellen, Zellverbände, Gewebe in ihrer natürlichen oder artifiziell konstruierten oder modifizierten Form umfasst.

Der Begriff "Zellen", wie hier verwendet, umfasst sowohl prokaryotische als auch eukaryotische Zellen, insbesondere Zellen von Bakterien und Archaebakterien, Pilzzellen, tierische und pflanzliche Zellen. Die Zellen können sowohl natürliche Zellen sein als auch Zellen, die in irgendeiner Weise, z.B. gentechnisch, modifiziert wurden. Diese Modifizierung kann insbesondere die Einführung zusätzlicher Zellbestandteile, (z.B. zusätzliches Genmaterial), den Austausch natürlicher Zellbestandteile durch entsprechende Bestandteile aus anderen Zellen oder künstliche Konstrukte, und die Deletion bestimmter Zellbestandteile, sowie Kombinationen davon umfassen.

Diese Materialien können insbesondere auch völlig synthetisch aufgebaute artifizielle Zellen und Vesikel umfassen, die Komponenten von Viren und Zellen, Aminosäuren, Peptide, Oligopeptide, Proteine, Enzyme, Nukleotide, Nukleoside, Nukleinsäuren (DNA,RNA), Ribozyme, Saccharide, Oligosaccharide, Polysaccharide, Lipide, Membranen und Membrankomponenten umfassen oder daraus bestehen.

In einer besonders bevorzugten Ausführungsform sind die Zellen lebende prokaryotische oder eukaryotische Zellen.

Vorzugsweise ist die Polymerkomponente des Gels durch Vernetzung von mindestens einer Monomerkomponente, die aus der Gruppe aus Acrylaten, Methacrylaten, Acrylamiden, Methacrylamiden, Styrolverbindungen, Vinylverbindungen, Allylverbindungen, Dienen, Epoxiden und deren Derivaten ausgewählt ist, gebildet.

Diese Derivate sind oder umfassen beispielsweise Konjugate von Acrylaten, Methacrylaten, Acrylamiden, Methacrylamiden, Styrolverbindungen, Vinylverbindungen, Allylverbindungen, Dienen, Epoxiden mit anderen Molekülen und Makromolekülen, insbesondere Polysachariden, z.B. Cellulose, Chitin, Alginate, Hyaluronsäure etc., Polyaminosacchariden, z.B. Chitosan; Proteinen, z.B. Collagen, Gelatine; Lipiden; anderen vernetzbaren Biopolymeren; Liposomen/Mizellen.

Die mit dem erfindungsgemäßen Verfahren erhältlichen Gele können sowohl Gelpartikel als auch flächige oder dreidimensionale Strukturen, letztere hier als "monolithische Strukturen" bezeichnet, einschließlich Membranen, umfassen.

In einer bevorzugten Ausführungsform sind die mit dem erfindungsgemäßen Verfahren erhältlichen Gele biologisch abbaubar und/oder physiologisch resorbierbar.

Ein weiterer Aspekt der vorliegenden Erfindung betrifft eine Materialzusammensetzung, die ein erfindungsgemäßes Gel umfasst.

Eine solche Materialzusammensetzung kann beispielsweise Füll- und Hilfsstoffe, einschließlich Nano- oder Mikropartikel oder -fasern, und/oder einen Träger oder eine Matrix auf oder in der sich das Gel befindet, umfassen.

Die erfindungsgemäßen Gele oder eine Materialzusammensetzung, welche diese Gele umfasst, eignen sich für eine Vielfalt von Einsatzgebieten.

Einige spezielle Anwendungen sind die Verwendung als Träger und Substrate für Zellen und Gewebe, beispielsweise als *in vitro* Modelle für zellbasierte Assays, z.B. beim Wirkstoffscreening, in Zell- und Gewebekulturen, als Träger für aktive Materialien in der Analytik, insbesondere Umweltanalytik und medizinischen Diagnostik, Katalysetechnik, Sensorik, als Enzymreaktor, bei der Sanierung und Aufbereitung von Wasser, Boden und Luft, als Extraktionsmedien, in der Implantologie, Chromatographie.

### Kurzbeschreibung der Figuren

Fig. 1 zeigt eine REM-Aufnahme eines Cryogels basierend auf 5% PEGMA/ 5% TEGDA.
Fig. 2 zeigt eine REM-Aufnahme eines entsprechenden Cryogels mit eingelagerten Hefezellen.
Fig. 3 zeigt eine REM-Aufnahme von Cryogelpartikeln, erhalten durch elektronenstrahlinduzierte Emulsionspolymerisation.
**Fig. 4** zeigt die relative Aktivität von Trypsinlösungen unter verschiedenen Puffer- und Temperaturbedingungen.
**Fig. 5** zeigt die relative Aktivität von Trypsinlösungen unter verschiedenen Bestrahlungs- und Temperaturbedingungen.
**Fig. 6** zeigt die relative Aktivität von Cryogel-Enzymreaktoren mit eingelagertem Trypsin bei verschiedenen Konzentrationen.
**Fig. 7** zeigt die relative Aktivität von Cryogel-Enzymreaktoren mit eingelagertem Trypsin bei verschiedenen pH-Werten.
**Fig. 8** zeigt die Vitalität von Hefezellen nach Elektronenbestrahlung bei verschiedenen Temperaturen, nachgewiesen durch spezifische Anfärbung mit Fluoreszenzfarbstoffen.
**Fig. 9** zeigt die Vitalität von eingelagerten Hefezellen nach Elektronenbestrahlung bei ca. -15 °C auf einer Agarplatte (Zellen wurden durch Stempelung mit dem Cryogel übertragen).
**Fig. 10** zeigt die Struktur von gelatine-haltigen Cryogelen;
   a: REM-Aufnahme eines gelatine-haltigen Cryogels

   b: EDX-Kartierung eines gelatine-haltigen Cryogels mit zusätzlich eingebautem Hydroxylapatit

Die folgenden Ausführungsbeispiele dienen zur näheren Erläuterung der Erfindung, ohne dieselbe jedoch auf die speziellen Parameter und Bedingungen der Beispiele zu beschränken.

### BEISPIEL 1

### Elektronenstrahlsynthese eines Cryogels auf Basis von (Meth)acrylaten in Eis

### Ausgangsmaterialien:

Polyethylenglykolmethacrylat (PEGMA) und Tetraethylenglykoldiacrylat (TEGDA; als Vernetzer), Millipore-grade Wasser 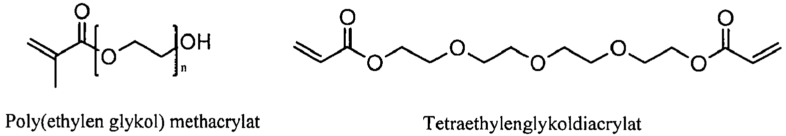
1. Herstellung der Polymerisationslösungen
   - Einwaage der Monomere und Vernetzer, z.B. 5 Gew.-% PEGMA, 5 Gew.-% TEGDA, 90 Gew.-% Wasser im Standardsystem (Anmerkung: durch Verwendung anderer Konzentrationen können Struktur und Morphologie der Cryogele verändert werden)
   - Überführen in Probengefäße (z.B. Eppendorf-Tubes, Zentrifugenröhrchen, Mikrotiterplatten oder Kapillarsäulen)
   - Lösen und Homogenisieren, Entgasen mit gasförmigem Stickstoff und Ultraschallbad
2. Einfrieren der Polymerisationslösungen
   - Einfrieren im Kryostat, 2 h, T = -20 °C im Standardsystem (Anmerkung: andere Temperaturen und Einfriergeschwindigkeiten ermöglichen Einfluss auf Struktur und Morphologie der Cryogele)
   - Überführung in Kühlbox zum Beschleuniger
3. Elektronenbestrahlung
   - Bestrahlung in Kühlbox bei ca. -15°C bis -20°C
   - Dosis D = 12 kGy in 3 kGy Schritten; Dauer: ca. 10 min (Anmerkung: höhere Dosis durch längere Bestrahlungszeit erhältlich, optimale Dosis variiert in Abhängigkeit von den Monomeren, Ursache unterschiedliche Reaktivität)
4. Auftauprozess + Spülen
   - Auftauen und gleichzeitiges Spülen mit Wasser (mehrmals)
   - Trocknen im VTS (fertig für die weitere Analyse und Bearbeitung)
5. Charakterisierung Ermittlung der chemischen Zusammensetzung mittels XPS (Nachweis von N) (siehe Tab. 1 in Beispiel 2)

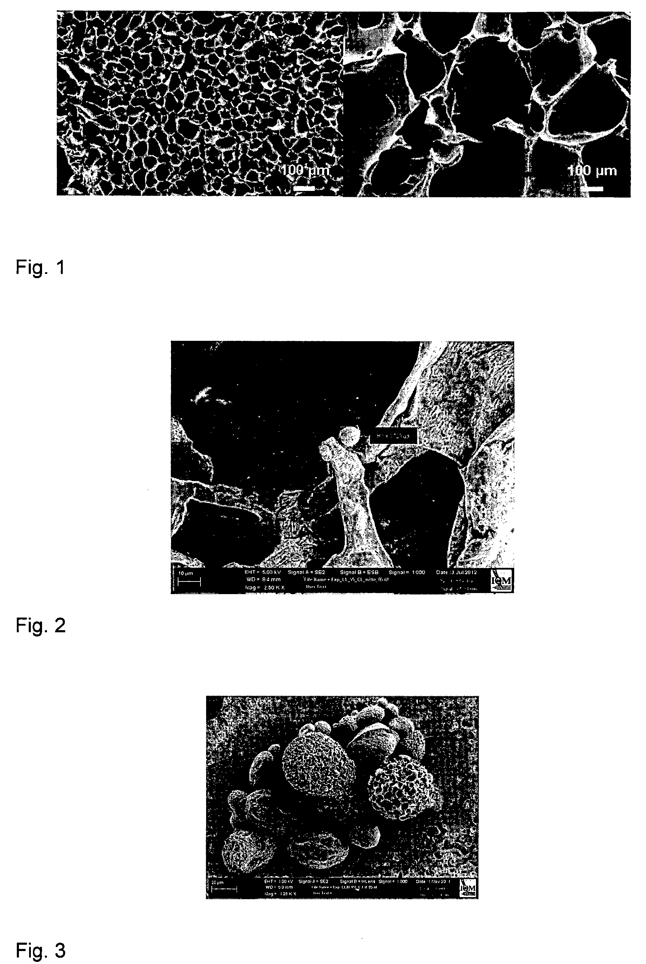

Fig. 1 zeigt eine rasterelektronenmikroskopische Aufnahme eines Cryogels basierend auf 5% PEGMA/ 5% TEGDA wie oben beschrieben.

### BEISPIEL 2

### Elektronenstrahlsynthese eines Cryogels auf Basis von (Meth)acrylaten und reaktiven Comonomeren in Eis

### Ausgangsmaterialien:

Analog Bsp. 1 (PEGMA, TEGDA) + Polyallylamin (PAAm) 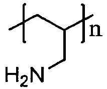 Polyallylamin besitzt im Gegensatz zu den eingesetzten (Meth(acrylat)monomeren keine Doppelbindung.
1. Herstellung der Polymerisationslösung
   - Einwaage der Monomere und Vernetzer (z.B. 5 Gew.-% PEGMA, 5 Gew.-% TEGDA, 5 % PAAm, 85 Gew.-% Wasser)
   - Weitere Schritte analog 1
2. -4. analog Beispiel 1
5. Charakterisierung
   - Chemische Zusammensetzung mittels XPS (Nachweis von N)

Tab. 1: Atomkonzentrationen und -verhältnisse von Standardcryogelen und NH₂-funktionalen Cryogelen

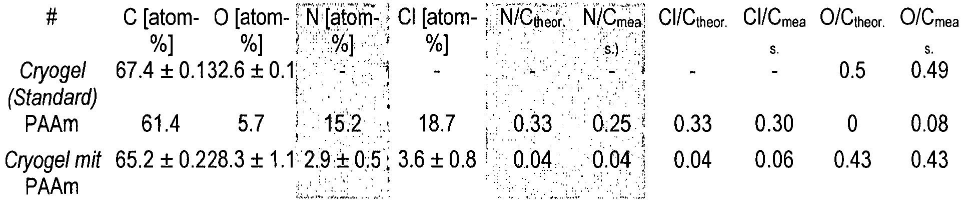

### BEISPIEL 3

### Elektronenbestrahlung von Trypsinlösungen und eingelagertem Trypsin bei verschiedenen Temperaturen und Überprüfung der Aktivität

### A: Elektronenbestrahlung von Trypsinlösungen

### Materialien:

Trypsin (Tn, gelöst in unterschiedlichen Puffern, z.B. Wasser, Hepes, verd. HCl.)
1. Herstellung Trypsinlösungen (c = 1-3 mg/ml)
2. einen Teil der Proben wie in Beispiel 1 einfrieren, den anderen Teil bei Raumtemperatur lagern
3. einen Teil der Proben wie in Beispiel 1 bei T = -15 °C bestrahlen, den anderen Teil bei Raumtemperatur
4. Auftauen und Überprüfung der Aktivität mit Verdau von L-BAPA - Überprüfung der Aktivität mit Hilfe einer bekannten Nachweisreaktion (Freisetzung von P-Nitroanilin durch Substratverdau bei Anwesenheit von Trypsin)
   - Substrat: N-benzoyl-L-arginin-p-Nitroanilid in 50 % DMSO und 50 % HEPES Puffer (pH 8, 100 mM HEPES, 200 mM NaCl und 20 mM CaCl₂)
   - Photometrische Messung der Freisetzung von p-Nitroanilin (λ = 405 nm)

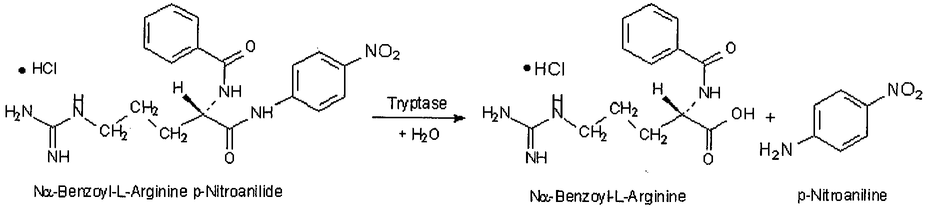

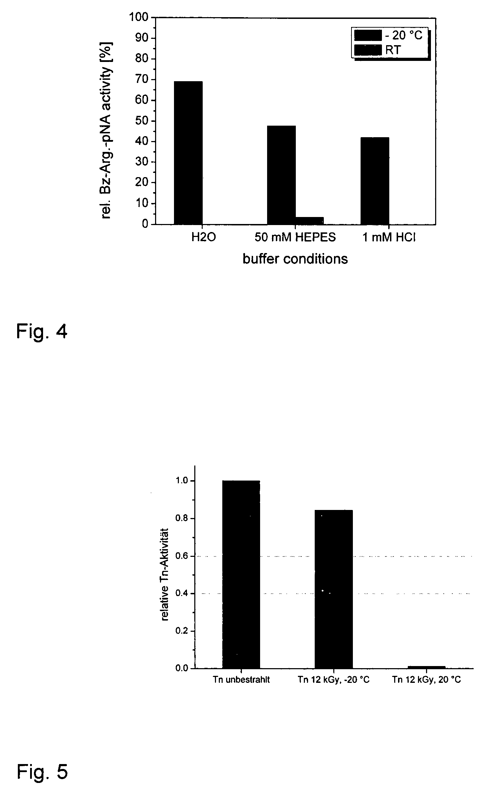

Fig. 4 zeigt die relative Aktivität von Trypsinlösungen unter verschiedenen Puffer- und Temperaturbedingungen.

Fig. 5 zeigt die relative Aktivität von Trypsinlösungen unter verschiedenen Bestrahlungs- und Temperaturbedingungen.

### B: Elektronenbestrahlung und in-situ Einlagerung von Trypsin in Cryogele und Überprüfung der Aktivität

### Materialien:

analog Beispiel 1 (PEGMA, TEGDA, Wasser) + Trypsin (Enzym)
1. Herstellung der Polymerisationslösung
   - Einwaage der Monomere, Vernetzer + Trypsin (Bsp. 5 Gew.-% PEGMA, 5 Gew.-% TEGDA, 3 mg/ml Trypsin, 90 Gew.- % Wasser)
   - Weitere Schritte analog Beispiel 1
2. -4. analog Beispiel 1
5. Überprüfung der Aktivität analog A

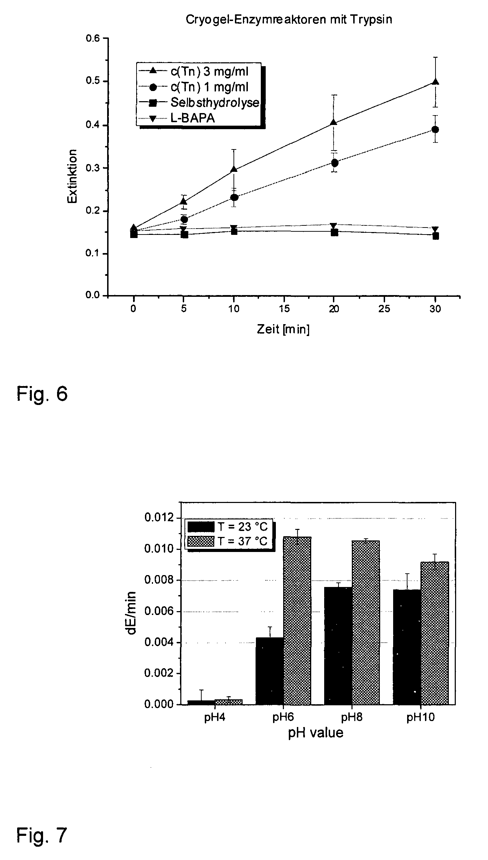

Fig. 6 zeigt die Ergebnisse einer Kinetikmessung:
Enzymaktivität von Tn-Cryogel-Reaktoren in Abhängigkeit von der Enzymkonzentration

Fig. 7 zeigt die Enzymaktivität von Tn-Cryogel-Reaktoren in Abhängigkeit von pH und Temperatur (präpariert in Mikrotiterplatten).

### BEISPIE1 4

### Elektronenbestrahlung von Hefezellen in Lösung/Dispersion und eingelagerten Hefezellen bei verschiedenen Temperaturen und Überprüfung der Aktivität

### A: Elektronenbestrahlung von Hefezellen in Lösung/Dispersion

### Materialien:

normale Bäckerhefe aus Supermarkt, 1mg/ml gelöst in PBS (Phosphatpuffer, pH 7,2)
1. Herstellung von Hefelösungen (c = 1mg/ml)
2. einen Teil der Proben wie in Beispiel 1 einfrieren, den anderen Teil bei Raumtemperatur lagern
3. einen Teil der Proben wie in Beispiel 1 bei T = -15 °C bestrahlen, den anderen Teil bei Raumtemperatur
4. Auftauen und Überprüfung der Morphologie und Vitalität der Zellen

Fig. 8 zeigt die Ergebnisse einer spezifischen Anfärbung vitaler und toter Hefezellen mit Fluoreszenzfarbstoffen, bestrahlt mit D = 12 kGy bei Raumtemperatur und bei T = -15 °C

### B: Elektronenbestrahlung und in-situ Einlagerung von Hefezellen in Cryogele

### Materialien:

normale Bäckerhefe aus Supermarkt, 1mg/ml gelöst in PBS PEGMA, TEGDA
1. Herstellung der Polymerisationsmischung
   - Einwaage der Monomere, Vernetzer + Hefe (Bsp. 5 Gew.- % PEGMA, 5 Gew.-% TEGDA, 1 mg/ml Hefe, 90 Gew.-% PBS)
2. Einfrieren analog Beispiel 1
3. Bestrahlen analog Beispiel 1
4. Auftauen und Spülen, sowie Überprüfung der Vitalität der Zellen

Fig. 2 zeigt die rasterelektronenmikroskopische Aufnahme eines Cryogels mit eingelagerten Hefezellen.

Fig. 9 zeigt die Vitalität von eingelagerten Hefezellen nach Elektronenbestrahlung bei ca. -15°C auf einer Agarplatte (Zellen wurden durch Stempelung mit dem Cryogel übertragen).

### BEISPIEL 5

### Elektronenstrahlsynthese von Cryogelpartikeln

### Materialien:

Polyethylenglykolmethacrylat (PEGMA) und Tetraethylenglykoldiacrylat (TEGDA), Millipore-grade Wasser, Span 80, Tween, Heptan
1. Herstellung der Polymerisationslösungen
• Herstellung einer Stammlösung von Span 80/Tween/Heptan 0,05 g/0,01 g/3,4 g - gespült mit N₂
• Einwaage der Monomere und Vernetzer entsprechend der folgenden Tabelle

| **Nr.** | **PEGMA [g]** | **TEGDA [g]** | **Wasser [µl]** |
|---|---|---|---|
| CL_30_PT_1 | 0,06 | 0,06 | 300 |
| CL_30_PT_2 | 0,06 | 0,03 | 300 |
| CL_30_PT_3 | 0,06 | 0,01 | 300 |

• Je 190 µl Mischung + 1,7 g Detergentienmischung
• Rühren mit Rührfisch (I)
2. Einfrieren der Polymerisationslösungen
- Einfrieren in flüssigen Stickstoff

3. Überführung in Kühlbox zum Beschleuniger
4. Elektronenbestrahlung
- Bestrahlung in flüssigem Stickstoff
- D = 30 kGy in 3 kGy Schritten

5. Auftauprozess + Spülen
- Zentrifugation
- Mehrmals spülen mit Wasser

6. Trocknen im VTS (fertig für die weitere Analyse und Bearbeitung)

Fig. 3 zeigt rasterelektronische Aufnahmen von Partikeln und Cryogelpartikeln, erhalten durch elektronenstrahlinduzierte Emulsionspolymerisation wie oben beschrieben.

### BEISPIEL 6

### Herstellung von Cryogelen mittels kurzwelliger UV-Strahlung

Eine Mischung aus 5% PEGMA, 5% TEGDA, 90% Wasser oder einer wässrigen Zellsuspension (alle %-Angaben in (w/w)) wird hergestellt und bei ca. 20°C Ausgangstemperatur im Tiefkühlschrank (ca. -20°C) oder flüssigem Stickstoff eingefroren (gegebenenfalls vor dem Einfrieren für 10 min mit Stickstoff entgasen). Zur Bestrahlung werden die Proben in eine thermostatierbare Kühlbox überführt und unter Stickstoff mit einer 172nm-VUV-Lampe (Hereaus Excimer Laborsystem, 2x20 mW/cm²) für .ca. 1-2 min bestrahlt. Anschließend wird die polymerisierte Probe aufgetaut und gewaschen.

### BEISPIEL 7

### Einbau eines nicht doppelbindungshaltigen Biopolymers in ein Cryogel auf Acrylatbasis

Als beispielhaftes Biopolymer wurde Gelatine verwendet.

### Durchführung:

Gelatine in Pulverform wurde in Konzentrationen bis 3 Gew.-% einer essigsauren, wässrigen Polyethylenglycol-Acrylatmischung zugesetzt und mit einem Kryostaten auf -20°C gebracht. Nach einer zweistündigen Einfrierzeit wurden die Proben mittels Elektronenbestrahlung mit einer Dosis von 12 kGy vernetzt. Nach der Bestrahlung wurden die Proben bei Raumtemperatur aufgetaut und mehrmals mit Wasser gespült.

### Ergebnisse:

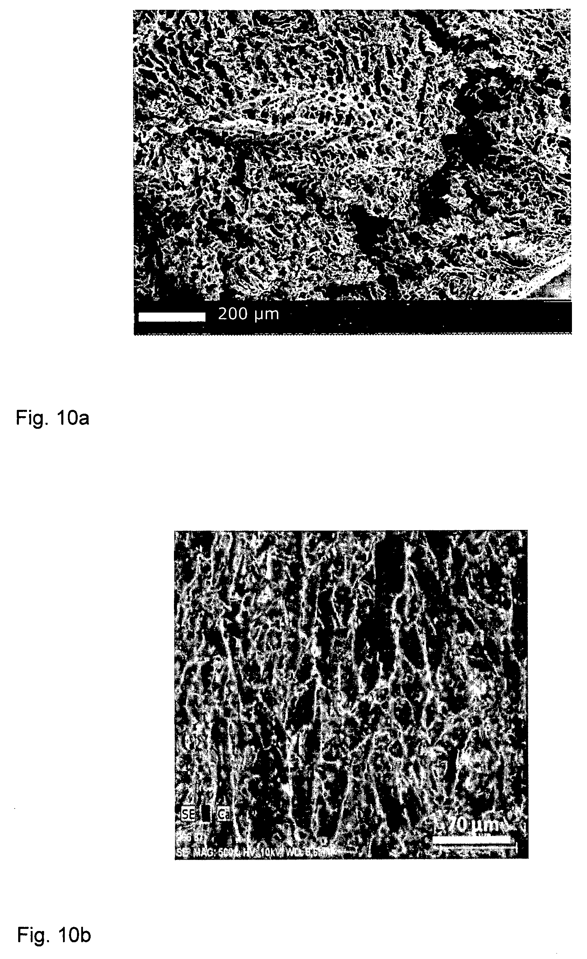

Die Synthese verlief erfolgreich. Mittels XPS gelang der Nachweis des Einbaus der Gelatine. In Abhängigkeit von der eingesetzten Gelatinemenge fanden sich bis zu ca. 90 % im Cryogel wieder (höchste Einbaurate bei ca. 2 % Gelatine-Ausgangskonzentration festgestellt), dabei war die Gelatine konzentrationsabhängig mehr oder weniger homogen im Cryogel verteilt. Bei diesen gelatinehaltigen Cryogelen handelt es sich um formstabile Materialien. Rasterelektronenmikroskopische Untersuchungen belegten die typische Cryogelmorphologie (Fig. 10a).

In einem weiteren Versuch wurden der obigen gelatinehaltigen Reaktionsmischung Hydroxylapatit-Nanopartikel (bis 5 Gew.-%) hinzugefügt. Hydroxylapatit ist ein Mineral, welches in der Biomineralisation (Knochenbildung) eine große Rolle spielt.

Die erhaltenen Cryogele wurden mit Wasser gespült und der Gehalt an Calcium mittels ICP-OES (optische Emissionsspektrometrie mittels induktiv gekoppelten Plasma) ermittelt. Die experimentellen Werte wurden mit den berechneten Gehalten an Ca verglichen. Die Ergebnisse in Tab. 1 belegen, dass die Partikel nahezu quantitativ in die Matrix eingelagert werden konnte. In Fig. 10b ist die Verteilung der Calciumpartikel im Cryogel mittels energiedispersiver Röntgenspektroskopie (EDX) dargestellt.

**Tab. 1: Vergleich der Ergebnisse der ICP-Analyse mit dem theoretischen Ca-Anteil**

| Probe | Theor. Ca-Anteil (Gew.-%) | Exp. Ca-Anteil (ICP-OES) |
|---|---|---|
| 1 | 5 | 4,9 ± 0,1 |
| 2 | 20 | 20,1 ± 0,6 |
| 3 | 35 | 34,9 ± 2,4 |

## Patentansprüche

1. Verfahren zur Herstellung von porösen Gelen, die Poren im Nano- bis Mikrometerbereich aufweisen, mit inkorporierten katalytisch oder biologisch aktiven Materialien, umfassend die folgenden Schritte:
a) Bereitstellen eines flüssigen Mediums, welches ein Lösungsmittel oder ein Lösungsmittelgemisch, das bei Abkühlung unter eine Phasenübergangstemperatur eine feste Phase mit amorphen und/oder kristallinen Strukturen ausbildet, mindestens eine durch Elektronenstrahlung oder kurzwellige UV-Strahlung im Wellenlängenbereich von 100 bis 250 nm vernetzbare Monomerkomponente, sowie mindestens ein katalytisch oder biologisch aktives Material in Lösung, Dispersion oder Emulsion umfasst und frei von Polymerisationsinitiatoren ist;
b) Abkühlen des flüssigen Mediums auf eine Temperatur unterhalb der Phasenübergangstemperatur des flüssigen Mediums;
c) Bestrahlen des abgekühlten Mediums von Schritt b) mit Elektronenstrahlung oder kurzwelliger UV-Strahlung im Wellenlängenbereich von 100 nm bis 250 nm mit einer ausreichenden Dosis, um eine Vernetzung der vernetzbaren Monomerkomponente(n) zu erreichen, wobei die amorphen oder kristallinen Strukturen der in Schritt b) gebildeten festen Phase als Matrize (Template) für das entstehende Netzwerk dienen;
d) Entfernen des Lösungsmittels oder Lösungsmittelgemischs, wodurch ein poröses Gel entsteht, dessen Poren eine durch die amorphen oder kristallinen Strukturen der in Schritt b) gebildeten festen Phase vorgegebene Größe und Form aufweisen.

2. Verfahren nach Anspruch 1, **dadurch gekennzeichnet, dass** das Lösungsmittel oder Lösungsmittelgemisch Wasser, ein organisches Lösungsmittel mit einem Phasenübergangspunkt im Bereich von -100°C bis + 100°C, eine ionische Flüssigkeit oder ein superkritisches Fluid umfasst oder daraus besteht.

3. Verfahren nach Anspruch 2, **dadurch kennzeichnet, dass** es die folgenden Schritte umfasst:
a) Bereitstellen eines flüssigen Mediums, welches als Lösungsmittel Wasser, mindestens eine durch Elektronenstrahlung oder kurzwellige UV-Strahlung im Wellenlängenbereich von 100 nm bis 250 nm vernetzbare Monomerkomponente, sowie mindestens ein katalytisch oder biologisch aktives Material in Lösung, Dispersion oder Emulsion umfasst;
b) Abkühlen des flüssigen Mediums auf eine Temperatur unterhalb des Gefrierpunkts, vorzugsweise im Bereich zwischen -15°C und -30°C,
c) Bestrahlen des abgekühlten, gefrorenen Mediums von Schritt b) mit Elektronenstrahlung oder kurzwelliger UV-Strahlung im Wellenlängenbereich von 100 nm bis 250 nm mit einer ausreichenden Dosis, um eine Vernetzung der vernetzbaren Monomerkomponente(n) zu erreichen;
d) Entfernen des Lösungsmittels.

4. Verfahren nach einem der Ansprüche 1-3, **dadurch gekennzeichnet, dass** die mindestens eine vernetzbare Monomerkomponente aus der Gruppe aus Acrylaten, Methacrylaten, Acrylamiden, Methacrylamiden, Styrolverbindungen, Vinylverbindungen, Allylverbindungen, Dienen, Epoxiden und deren Derivaten ausgewählt ist.

5. Verfahren nach einem der Ansprüche 1-4, **dadurch gekennzeichnet, dass** die vernetzbare Monomerkomponente(n) in einer Gesamtmenge von 1-20 Gew.-%, vorzugsweise 1-15 Gew.-%, insbesondere 4-10 Gew.-%, in dem flüssigen Medium vorliegt/en.

6. Verfahren nach einem der Ansprüche 1-5 zur Herstellung von Gelpartikeln, **dadurch gekennzeichnet, dass** es eine Emulsionspolymerisation von in dem jeweiligen Lösungsmittel unlöslichen vernetzbaren Monomerkomponenten umfasst.

7. Verfahren nach einem der Ansprüche 1-6, **dadurch gekennzeichnet, dass** das flüssige Medium ferner eine Reporterkomponente umfasst, welche das Erreichen eines gewünschten Vernetzungsgrads durch eine nachweisbare Änderung eines physikalischen Parameters, vorzugsweise eines optischen Parameters wie Farbe oder Fluoreszenz, anzeigt.

8. Poröses Gel, erhältlich mit dem Verfahren nach einem der Ansprüche 1-7 und umfassend eine vernetzte Polymerkomponente und inkorporierte katalytisch oder biologisch aktive Materialien, **dadurch gekennzeichnet, dass** es frei von Polymerisationsinitiatoren und organischen Lösungsmitteln ist und eine mittlere Porengröße von 30-100 µm, vorzugsweise 35-100 µm, aufweist.

9. Poröses Gel nach Anspruch 8 oder Verfahren nach einem der Ansprüche 1-7, **dadurch gekennzeichnet, dass** die katalytisch oder biologisch aktiven Materialien aus der Gruppe ausgewählt sind, die Katalysatoren, physiologische oder pharmazeutische Wirkstoffe, Aminosäuren, Peptide, Oligopeptide, Proteine, Enzyme, Nukleotide, Nukleoside, Nukleinsäuren, Ribozyme, Saccharide, Oligosaccharide, Polysaccharide, Lipide, Membranen und Membrankomponenten, intra- und interzelluläre Regulationsstoffe, Viren, Zellbestandteile, Zellorganellen, Zellen, Zellverbände und Gewebe umfasst.

10. Gel oder Verfahren nach Anspruch 9, **dadurch gekennzeichnet, dass** die biologisch aktiven Materialien natürliche Zellen oder Zellen, welche durch die Einführung zusätzlicher Zellbestandteile, den Anstausch natürlicher Zellbestandteile durch entsprechende Bestandteile aus anderen Zellen oder künstliche Konstrukte, die Deletion bestimmter Zellbestandteile, oder Kombinationen davon modifiziert wurden, oder synthetisch aufgebaute artifizielle Zellen und Vesikel, die Komponenten von Viren und Zellen, Aminosäuren, Peptide, Oligopeptide, Proteine, Enzyme, Nukleotide, Nukleoside, Nukleinsäuren (DNA,RNA), Ribozyme, Saccharide, Oligosaccharide, Polysaccharide, Lipide, Membranen und Membrankomponenten umfassen oder daraus bestehen, umfassen.

11. Gel oder Verfahren nach Anspruch 10, **dadurch gekennzeichnet, dass** die biologisch aktiven Materialien lebende prokaryotische oder eukaryotische Zellen sind.

12. Gel nach einem der Ansprüche 8-11, **dadurch gekennzeichnet, dass** die Polymerkomponente durch Vernetzung von mindestens einer Monomerkomponente, die aus der Gruppe aus Acrylaten, Methacrylaten, Acrylamiden, Methacrylamiden, Styrolverbindungen, Vinylverbindungen, Allylverbindungen, Dienen, Epoxiden und deren Derivaten ausgewählt ist, gebildet ist.

13. Gel nach Anspruch 12 oder Verfahren nach Anspruch 4, **dadurch gekennzeichnet, dass** die Derivate Konjugate von Acrylaten, Methacrylaten, Acrylamiden, Methacrylamiden, Styrolverbindungen, Vinylverbindungen, Allylverbindungen, Dienen, Epoxiden mit anderen Molekülen und Makromolekülen, insbesondere Polysachariden, Polyaminosacchariden, Proteinen, Lipiden, Hyaluronsäure, anderen vernetzbaren Biopolymeren, Liposomen/Mizellen darstellen oder umfassen.

14. Materialzusammensetzung, umfassend ein Gel nach einem der Ansprüche 8-13 sowie gegebenenfalls ferner Füll- und Hilfsstoffe, einschließlich Nano- oder Mikropartikel oder -fasern, und/oder einen Träger oder eine Matrix auf oder in der sich das Gel befindet.

15. Verwendung der Gele nach einem der Ansprüche 8-13 oder der Materialzusammensetzung nach Anspruch 14 als Träger und Substrate für Zellen und Gewebe, beispielsweise als *in vitro* Modelle für zellbasierte Assays, z.B. beim Wirkstoffscreening, in Zell- und Gewebekulturen, als Träger für aktive Materialien in der Analytik, insbesondere Umweltanalytik und medizinischen Diagnostik, Katalysetechnik, Sensorik, als Enzymreaktor, bei der Sanierung und Aufbereitung von Wasser, Boden und Luft, als Extraktionsmedien, in der Implantologie, Chromatographie.

## Claims

1. A method for the preparation of porous gels, having pores in the nanometre to micrometre range, with incorporated catalytically or biologically active materials, comprising the following steps:
a) providing a liquid medium which comprises a solvent or a solvent mixture that, on cooling to below a phase transition temperature, forms a solid phase with amorphous and/or crystalline structures, at least one monomer component cross-linkable by means of electron radiation or short-wave UV radiation in the wavelength ranging from 100 to 250 nm, as well as at least one catalytically or biologically active material in solution, dispersion or emulsion, and is free of polymerisation initiators;
b) cooling of the liquid medium to a temperature below the phase transition temperature of said liquid medium;
c) irradiating the cooled medium from step b) with electron radiation or short-wave UV radiation in the wavelength ranging from 100 to 250 nm with a dose sufficient to achieve cross-linking of the cross-linkable monomer component(s), wherein the amorphous or crystalline structures of the solid phase formed in step b) serve as a template for the emerging network;
d) removing the solvent or solvent mixture, whereby a porous gel is formed, the pores of which have a size and shape predetermined by the amorphous or crystalline structures of the solid phase formed in step b).

2. The method according to claim 1, **characterised in that** the solvent or solvent mixture comprises or consists of water, an organic solvent with a phase transition point ranging from -100°C to + 100°C, an ionic liquid or a supercritical fluid.

3. The method according to claim 2, **characterised in that** it comprises the following steps:
a) providing a liquid medium which comprises water as the solvent, at least one monomer component cross-linkable by means of electron radiation or short-wave UV radiation in the wavelength ranging from 100 nm to 250 nm, as well as at least one catalytically or biologically active material in solution, dispersion or emulsion;
b) cooling of the liquid medium to a temperature below freezing point, preferably in the range between -15°C and -30°C,
c) irradiating the cooled, frozen medium from step b) with electron radiation or short-wave UV radiation in the wavelength ranging from 100 nm to 250 nm with a dose sufficient to achieve cross-linking of the cross-linkable monomer component(s);
d) removing the solvent.

4. The method according to one of claims 1-3, **characterised in that** the at least one cross-linkable monomer component is selected from the group of acrylates, methacrylates, acrylamides, methacrylamides, styrene compounds, vinyl compounds, allyl compounds, dienes, epoxides and their derivatives.

5. The method according to one of claims 1-4, **characterised in that** the cross-linkable monomer component(s) are present in the liquid medium in a total quantity of 1-20 wt%, preferably 1-15 wt%, in particular 4-10 wt%.

6. The method according to one of claims 1-5 for the preparation of gel particles, **characterised in that** it comprises an emulsion polymerisation of cross-linkable monomer components insoluble in the respective solvent.

7. The method according to one of claims 1-6, **characterised in that** the liquid medium further comprises a reporter component which indicates the reaching of a desired degree of cross-linking by means of a detectable change in a physical parameter, preferably an optical parameter such as colour or fluorescence.

8. A porous gel, obtainable by the method according to one of claims 1-7 and comprising a cross-linked polymer component and incorporated catalytically or biologically active materials, **characterised in that** it is free of polymerisation initiators and organic solvents and has an average pore size of 30-100 µm, preferably 35-100 µm.

9. The porous gel according to claim 8 or method according to one of claims 1-7, **characterised in that** the catalytically or biologically active materials are selected from the group which comprises catalysts, physiologically or pharmaceutically active agents, amino acids, peptides, oligopeptides, proteins, enzymes, nucleotides, nucleosides, nucleic acids, ribozymes, saccharides, oligosaccharides, polysaccharides, lipids, membranes and membrane components, intra- and intercellular regulation substances, viruses, cell components, cell organelles, cells, cell aggregates and tissues.

10. The gel or method according to claim 9, **characterised in that** the biologically active materials comprise natural cells or cells which were modified by the introduction of additional cell components, by the replacement of natural cell components with corresponding components from other cells or synthetic constructs, by the deletion of specific cell components, or by combinations thereof, or synthetically constructed artificial cells and vesicles which comprise or consist of components of viruses and cells, amino acids, peptides, oligopeptides, proteins, enzymes, nucleotides, nucleosides, nucleic acids (DNA, RNA), ribozymes, saccharides, oligosaccharides, polysaccharides, lipids, membranes and membrane components.

11. The gel or method according to claim 10, **characterised in that** the biologically active materials are living prokaryotic or eukaryotic cells.

12. The gel according to one of claims 8-11, **characterised in that** the polymer component is formed by cross-linking of at least one monomer component which is selected from the group of acrylates, methacrylates, acrylamides, methacrylamides, styrene compounds, vinyl compounds, allyl compounds, dienes, epoxides and their derivatives.

13. The gel according to claim 12 or method according to claim 4, **characterised in that** the derivatives constitute or comprise conjugates of acrylates, methacrylates, acrylamides, methacrylamides, styrene compounds, vinyl compounds, allyl compounds, dienes, epoxides with other molecules and macromolecules, in particular polysaccharides, polyaminosaccharides, proteins, lipids, hyaluronic acid, other cross-linkable biopolymers, liposomes/micelles.

14. A material composition, comprising a gel according to one of claims 8-13 and optionally further fillers and auxiliary substances, including nano- or microparticles or nano- or microfibres, and/or a carrier or a matrix on or in which the gel is located.

15. Use of the gels according to one of claims 8-13 or the material composition according to claim 14 as carriers and substrates for cells and tissues, for example as *in vitro* models for cell-based assays, e.g. during drug screening, in cell and tissue cultures, as carriers for active materials in analysis, particularly environmental analysis and medical diagnosis, catalysis technology, sensor technology, as an enzyme reactor, in the remediation and treatment of water, soi and air, as extraction media, in implantology, chromatography.

## Revendications

1. Procédé servant à fabriquer des gels poreux, qui présentent des pores relevant de la plage nanométrique à micrométrique, comprenant des matériaux incorporés catalytiquement ou biologiquement actifs, comprenant les étapes suivantes consistant à :
a) mettre à disposition un milieu liquide, qui comprend un solvant ou un mélange de solvants, qui réalise, lors du refroidissement à une température inférieure à une température de transition de phase, une phase solide comprenant des structures amorphes et/ou cristallines, au moins un composant monomère réticulable par un rayonnement électronique ou par un rayonnement UV à ondes courtes relevant de la plage de longueurs d'onde allant de 100 à 250 nm, ainsi qu'au moins un matériau catalytiquement ou biologiquement actif sous la forme d'une solution, d'une dispersion ou d'une émulsion et qui est sans initiateurs de polymérisation ;
b) refroidir le milieu liquide à une température inférieure à la température de transition de phase du milieu liquide ;
c) exposer le milieu refroidi de l'étape b) à un rayonnement électronique ou à un rayonnement UV à ondes courtes relevant de la plage de longueurs d'onde allant de 100 nm à 250 nm, à une dose suffisante pour atteindre une réticulation du/des composants(s) réticulable(s), dans lequel les structures amorphes ou cristallines de la phase solide formée à l'étape b) servent de matrices (template) pour le réseau se formant ;
d) éliminer le solvant ou le mélange de solvants ce qui permet de créer un gel poreux, dont les pores présentent une taille et une forme prédéfinies par les structures amorphes ou cristallines de la phase solide formée à l'étape b).

2. Procédé selon la revendication 1, **caractérisé en ce que** le solvant ou le mélange de solvants comprend de l'eau, un solvant organique présentant un point de transition de phase situé dans la plage allant de -100°C à +100°C, un liquide ionique ou un fluide supercritique ou est constitué de ces éléments.

3. Procédé selon la revendication 2, **caractérisé en ce qu'**il comprend les étapes suivantes consistant à :
a) mettre à disposition un milieu liquide, qui comprend en tant que solvant de l'eau, au moins un composant monomère réticulable par un rayonnement électronique ou par un rayonnement UV à ondes courtes situé dans la plage de longueurs d'onde allant de 100 nm à 250 nm, ainsi qu'au moins un matériau catalytiquement ou biologiquement actif sous la forme d'une solution, d'une dispersion ou d'une émulsion ;
b) refroidir le milieu liquide à une température inférieure au point de congélation, de préférence située dans la plaque comprise entre -15°C et -30°C ;
c) exposer le milieu refroidi congelé de l'étape b) à un rayonnement électronique ou à un rayonnement UV à ondes courtes situé dans la plage de longueurs d'onde allant de 100nm à 250nm, avec une dose suffisante pour atteindre une réticulation du/des composant(s) monomère(s) réticulable(s) ;
d) éliminer le solvant.

4. Procédé selon l'une quelconque des revendications 1 à 3, **caractérisé en ce que** l'au moins un composant monomère réticulable est choisi parmi le groupe composé d'acrylates, de méthacrylates, d'acrylamides, de méthacrylamides, de composés styréniques, de composés vinyliques, de composés allyliques, de diènes, d'époxydes et de leurs dérivés.

5. Procédé selon l'une quelconque des revendications 1 à 4, **caractérisé en ce que** le/les composé(s) monomère(s) réticulable(s) sont présents dans le milieu fluide en une quantité totale de 1 à 20 % en poids, de préférence de 1 à 15% en poids, en particulier de 4 à 10% en poids.

6. Procédé selon l'une quelconque des revendications 1 à 5 servant à fabriquer des particules de gel, **caractérisé en ce que** le procédé comprend une polymérisation d'émulsion de composants monomères réticulables non solubles dans le solvant respectif.

7. Procédé selon l'une quelconque des revendications 1 à 6, **caractérisé en ce que** le milieu fluide comprend en outre un composant rapporteur, qui indique l'obtention d'un degré de réticulation souhaité par une modification pouvant être établie d'un paramètre physique, de préférence d'un paramètre visuel tel que la couleur ou la fluorescence.

8. Gel poreux, pouvant être obtenu à l'aide du procédé selon l'une quelconque des revendications 1 à 7 et comprenant un composant polymère réticulé et des matériaux incorporés catalytiquement ou biologiquement actifs, **caractérisé en ce que** ledit gel est sans initiateur de polymérisation et sans solvant organique, et **en ce qu'**il présente une taille de pores moyenne de 30 à 100 µm, de préférence de 35 à 100 µm.

9. Gel poreux selon la revendication 8 ou procédé selon l'une quelconque des revendications 1 à 7, **caractérisé en ce que** les matériaux catalytiquement ou biologiquement actifs sont choisis parmi le groupe comprenant des catalyseurs, des principes actifs physiologiques ou pharmaceutiques, des acides aminés, des peptides, des oligopeptides, des protéines, des enzymes, des nucléotides, des nucléosides, des acides nucléiques, des ribozymes, des saccharides, des oligosaccharides, des polysaccharides, des lipides, des membranes et des composants membranaires, des substances de régulation intra- et intercellulaires, des virus, des constituants cellulaires, des organelles cellulaires, des cellules, des amas cellulaires et des tissus.

10. Gel ou procédé selon la revendication 9, **caractérisé en ce que** les matériaux biologiquement actifs comprennent des cellules naturelles ou des cellules, qui ont été modifiées par l'introduction de constituants cellulaires supplémentaires, par le remplacement de constituants cellulaires naturels par des constituants correspondants composés d'autres cellules ou d'hybrides synthétiques, par la délétion de certains constituants cellulaires, ou par une combinaison de ces derniers, ou des cellules artificielles à structure synthétique et des vésicules, qui comprennent des composants de virus et de cellules, des acides aminés, des peptides, des oligopeptides, des protéines, des enzymes, des nucléotides, des nucléosides, des acides nucléiques (ADN, ARN), des ribozymes, des saccharides, des oligosaccharides, des polysaccharides, des lipides, des membranes et des composants membranaires, ou qui sont constitués de ces éléments.

11. Gel ou procédé selon la revendication 10, **caractérisé en ce que** les matériaux biologiquement actifs sont des cellules vivantes procaryotes ou eucaryotes.

12. Gel selon l'une quelconque des revendications 8 à 11, **caractérisé en ce que** le composant polymère est formé par réticulation d'au moins un composant monomère qui est choisi parmi le groupe composé d'acrylates, de méthacrylates, d'acrylamides, de méthacrylamides, de composés styréniques, de composés vinyliques, de composés allyliques, de diènes, d'époxydes et de leurs dérivés.

13. Gel selon la revendication 12 ou procédé selon la revendication 4, **caractérisé en ce que** les dérivés constituent ou comprennent des conjugués d'acrylates, de méthacrylates, d'acrylamides, de méthacrylamides, de composés styréniques, de composés vinyliques, de composés allyliques, de diènes, d'époxydes combinés à d'autres molécules et macromolécules, en particulier à des polysaccharides, des polyaminosaccharides, des protéines, des lipides, de l'acide hyaluronique, à d'autres biopolymères réticulables, des liposomes/micelles.

14. Composition de matériaux, comprenant un gel selon l'une quelconque des revendications 8 à 13, ainsi que le cas échéant en outre des charges et des matières auxiliaires, y compris des nano- ou microparticules ou des nano- ou microfibres, et/ou un support ou une matrice, sur lequel/laquelle ou dans lequel/laquelle se trouve le gel.

15. Utilisation des gels selon l'une quelconque des revendications 8 à 13 ou de la composition de matériaux selon la revendication 14 en tant que supports et substrats pour des cellules et des tissus, par exemple en tant que modèles in vitro pour des réseaux à base de cellules, par exemple lors du balayage de principe actif, dans des cultures de cellules et de tissus, en tant que supports pour des matériaux actifs dans l'analytique, en particulier dans l'analytique environnementale et dans le diagnostic médical, dans la technique de catalyse, dans la technique de détection, en tant que réacteur enzymatique, lors de l'assainissement et de la préparation d'eau, de sol et d'air, en tant que milieux d'extraction, en implantologie, en chromatographie.
